# EUROPEAN PATENT APPLICATION

(11) **EP 1 637 073 A1**
(43) Date of publication of application: **22.03.2006**
(21) Application number: 05020022.9
(22) Date of filing: 14.09.2005
(51) Int. Cl.: A61B 5/022

(54) **Sphygmomanometer**

(30) Priority: 15.09.2004 JP 2004268175
(71) Applicant: Omron Healthcare Co., Ltd., Kyoto 615-0084 (JP)
(72) Inventor: Hiroshi, Kishimoto Omron Healthcare Co., Ltd, Ukyo-ku Kyoto-shi Kyoto 615-0084 (JP); Yoshihiko, Sano Omron Healthcare Co., Ltd, Ukyo-ku Kyoto-shi Kyoto 615-0084 (JP); Hiromichi, Karo Omrin Healthcare Co., Ltd, Ukyo-ku Kyoto-shi Kyoto 615-0084 (JP)
(74) Representative: Kilian, Helmut

(57) **Abstract**

The housing employed in a sphygmomanometer includes a first housing (20) and a second housing (30). A cuff band (10) has a bending portion (B) that is bent inwardly. The bending portion (B) marks the division between the first housing (20) provided at one end and the second housing (30) provided at the other end on the outer circumferential surface of the cuffband (10). Accordingly, the entire volume can be changed significantly between a state of measurement and a state of storage. A sphygmomanometer having a structure that allows the storage capability and portability to be improved can be provided.

## Description

### BACKGROUND OF THE INVENTION

### Field of the Invention

The present invention relates to a sphygmomanometer, and a structure of a sphygmomanometer in association with storage capability and portability.

### Description of the Background Art

For everyday use of a sphygmomanometer of the type that has a cuff band wrapped around the upper arm or wrist of a human body to measure blood pressure, storage capability and portability of the main unit of the manometer and the cuff band are required to allow the sphygmomanometer to be carried around and measure the blood pressure irrespective of when and where measurement is conducted.

The sphygmomanometer disclosed in Document 1 (Japanese Patent Laying-Open No. 2003-299626) has a switchable configuration between a state of measurement in which the cuff band for obstructing blood pressure and the manometer main unit attain an overlapping posture and a state of storage in which the cuff band and the manometer main unit are juxtaposed in order to achieve a thin and compact configuration to facilitate portability. An integrated structure of the cuff band and manometer main unit is employed in such a sphygmomanometer. Document 2 (Japanese Patent Laying-Open No. 06-105813) discloses a hemodynamometer including a cuff band having concaves formed at the outer surface of a clip plate, and a hinge for rollup to allow inward folding and preventing excessive spreading outer than the circular cross section.

Document 3 (Japanese Utility Model Laying-Open No. 05-078203) discloses a sphygmomanometer employing a housing having a plurality of divided rigid bodies coupled through a flexible member in the circumferential direction of the cuff band to allow the curvature of the housing to be varied to improve attachability. Document 4 (Japanese Patent Laying-Open No. 09-285453) discloses a wristwatch type sphygmomanometer employing a structure in which the overall configuration is rendered thin by arranging the wristwatch portion and the blood pressure measurement portion perpendicular to the circumferential direction of the cuffband, and arranging the pump in the circumferential direction to improve attachability.

Document 5 (Japanese Patent Laying-Open No. 11-056796) discloses a hemomanometer employing a structure having a display arranged at the surface of the main unit component and an operation switch arranged at the side to allow the entire surface to be used as the area for arranging the display, and in which the thickness of the operation switch does not affect the entire thickness. Document 6 (Japanese Patent Laying-Open No. 06-114015) discloses a sphygmomanometer employing a structure in which a pair of protruding storage units having a shape in accordance with the outer circumferential plane of a squeezing band is arranged at the left and right lower sides of the main unit with a heavy-weighed object inside. Document 7 (Japanese Patent Laying-Open No. 11-042217) discloses a sphygmomanometer employing a structure in which the batteries are arranged in divided groups at respective ends of the main unit, and at the same height when viewed from the cuff band.

From the standpoint of storage capability, there is no great change in the entire volume between the state of measurement and the state of storage in which the form of the main unit and/or configuration of the curled elastic member portion is altered according to the respective structures in the conventional devices set forth above. It can be said that the storage capability is not of a desirable level. Even if the main unit is made thin to improve the attachability, the storage capability cannot be improved if the shape of the curled elastic member portion is the same. If the curled elastic member is made smaller to reduce the storage space or if the curled elastic member itself is made of a pliable material, the problem of the squeezing force of the cuff band and/or attachability being impaired may occur.

It is to be noted that, even if the internal components are reduced in size and the components are arranged more densely, the shape of the main unit will depend on the shape of the battery as long as the main unit has the battery disposed therein since the shape of the battery cannot be changed. There is a limit in reducing the size and thickness of the main body.

### SUMMARY OF THE INVENTION

The present invention aims to solve the problem of the form of a sphygmomanometer not being compact enough in a stored state, less vulnerable to improvement in storage capability and portability. Therefore, an object of the present invention is to provide a sphygmomanometer employing a structure that allows the storage capability and portability to be improved by varying the entire volume significantly between a state of measurement and a state of storage.

According to an aspect of the present invention, a sphygmomanometer includes a housing in which an apparatus for blood pressure measurement is stored, and a cuff band supporting the housing, and to be wrapped around a subject. The housing includes a first housing and a second housing. The cuffband includes a bending portion that bends. The bending portion marks a division between the first housing provided at one end and the second housing provided at the other end on an outer circumferential surface of the cuff band.

In accordance with the sphygmomanometer of the present invention, the housing in which an apparatus for blood pressure measurement is stored is divided into a first housing and the second housing between which a bending portion of the cuff band is located. Therefore, the cuff band can be bent sufficiently about the bending portion. As a result, a structure can be achieved in which the housing and the cuff band can be folded sufficiently, allowing improvement of the storage capability. Further, portability can also be improved by reducing the size of the storage case of the sphygmomanometer in a stored state.

Since the cuff band can be folded about the bending point, the storage capability can be improved even in the case where a cuff band incorporating a relatively rigid curled elastic member is employed to facilitate a firm fit of the cuff band on the subject at the time of attachment. Since it is not necessary to reduce the size of the cuff band or use a pliable cuff band, the squeezing performance and attachability of the cuff band will not be degraded. By virtue of a structure in which the main unit of the blood pressure measurement apparatus is stored in the first housing and the power supply is stored in the second housing, the first housing can be made thin, independent of the size of the power supply, while the size (thickness) of the second housing depends on the size of the power supply that is a battery or the like. Thus, the storage capability and portability can be further improved.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a plan view of a sphygmomanometer according to a first embodiment.
Fig. 2 is a side view of the sphygmomanometer of the first embodiment (only the cuff band is illustrated in cross section).
Fig. 3 is a sectional view of the sphygmomanometer of Fig. 1 in the direction of the arrow of III-III.
Fig. 4 is a side view of a structure of a curled elastic member of a cuff band employed in the sphygmomanometer of the first embodiment.
Fig. 5 is a side view of the curled elastic member of Fig. 4 in a broadened state.
Fig. 6 is a side view of the sphygmomanometer of the first embodiment attached to a subject.
Fig. 7 is a sectional view of the sphygmomanometer of the first embodiment attached to a subject.
Fig. 8 represents the sphygmomanometer of the first embodiment in a stored state in a case.
Fig. 9 is a side view of a sphygmomanometer according to a second embodiment in a basic form (only the cuff band is illustrated in cross section).
Fig. 10 is a side view of the sphygmomanometer of the second embodiment in an attached state (only the cuff band is illustrated in cross section).
Fig. 11 is a side view of a sphygmomanometer according to a third embodiment in a basic form (only the cuff band is illustrated in cross section).
Fig. 12 is a side view of the sphygmomanometer of the third embodiment in an attached state (only the cuff band is illustrated in cross section).

### DESCRIPTION OF THE PREFERRED EMBODIMENTS

A structure of a sphygmomanometer according to respective embodiments of the present invention will be described hereinafter with reference to the drawings. In each embodiment, the same or corresponding elements have the same reference characters allotted, and description thereof will not be repeated.

### First Embodiment

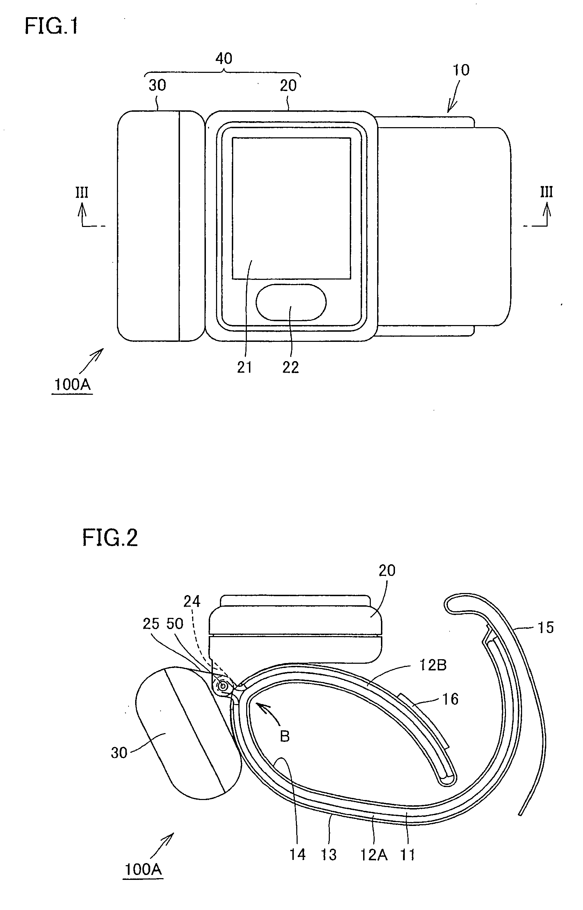

A structure of a sphygmomanometer 100A according to a first embodiment of the present invention will be described with reference to Figs. 1-5.

### Cuff Band 10

Referring to Figs. 1-3, sphygmomanometer 100A includes a housing 40 in which an apparatus for blood pressure measurement is stored, and a cuff band 10 supporting housing 40, and to be wrapped around a subject 1. Cuff band 10 includes an air bladder 11 that is inflated with air to apply pressure on the artery of a subject, and a curled elastic member 12 to maintain substantially an arc shape in cross section to facilitate the attachment around a subject (the wrist in Fig. 2). Air bladder 11 and curled elastic member 12 are covered by an outer texture 13 and an inner texture 14. A hook-and-loop fastener 16 is provided on the outer surface at one end of external texture 13. A hook-and-loop fastener 15 for adjustment is attached on the inner surface at the other end of outer texture 13. Hook and loop fastener 15 functions to adjust the contracting intensity and wrapping length of cuff band 10 around wrist 1.

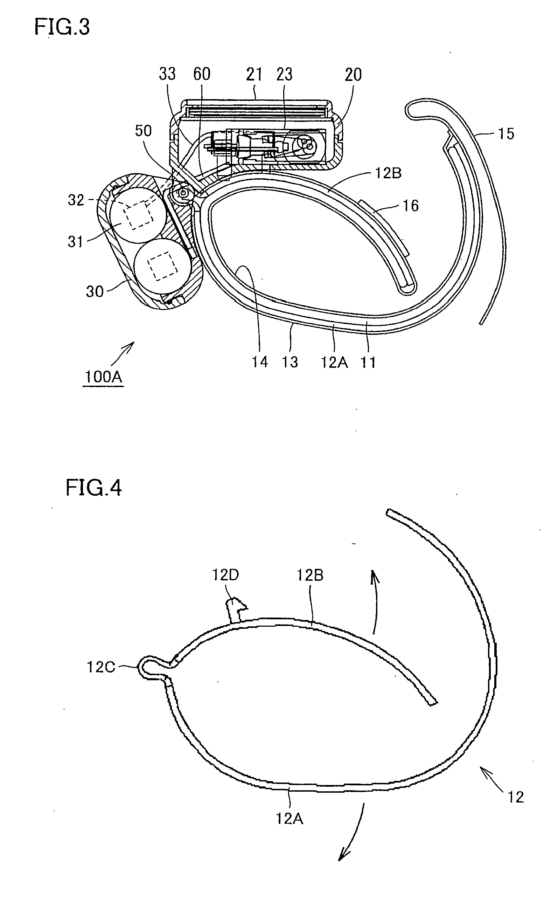

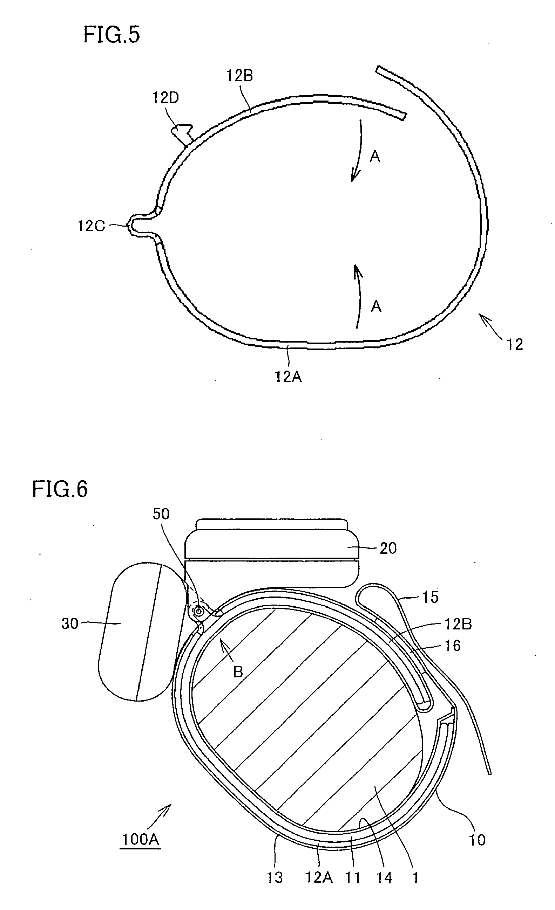

Curled elastic member 12 has a shape maintaining substantially an arc as a whole, as shown in Figs. 4 and 5. Curled elastic member 12 is formed of a resilient member such as flexible resin, taking a basic form of being folded inwardly (state of molded shape). Curled elastic member 12 includes a hinge 12C molded in a U symbol shape, and also a first curvature portion 12A and a second curvature portion 12B that are broadened towards an opening state with hinge 12C marking the division. One end of first curvature portion 12A overlaps with one end of second curvature portion 12B. A stopper 12D is provided on the outer circumferential surface of second curvature portion 12B. This stopper 12D engages with a hole provided at the bottom of first housing 20 that will be described afterwards to support first housing 20 in a detachable manner with respect to cuff band 10. A similar stopper can be provided on the outer circumferential surface of first curvature portion 12A to allow second housing 30 to be supported with respect to cuff band 10 in a detachable manner.

Since curled elastic member 12 of the present embodiment attains a stable state with the inwardly folded state as the basic form, the broadening of first and second curvature portions 12A and 12B outwardly, as shown in Fig. 5, causes generation of force towards the inner side (the force in the direction of arrow A in Fig. 5) by the resilience of curled elastic member 12 per se. Alternatively, a structure can be employed in which the broadened state of curvature portions 12A and 12B shown in Fig. 5 is taken as the basic form corresponding to a stable posture (state of molded shape) to generate force towards a broadening state by the resilience of curled elastic member 12 per se when in an inwardly folded state, as shown in Fig. 4.

In accordance with curled elastic member 12 set forth above, cuff band 10 has a bending portion B formed at hinge 2C of curled elastic member 12. By molding curled elastic member 12 out of a resilient member and deforming the shape of curled elastic member 12 per se (transition from the state of Fig. 5 to the state of Fig. 4, or from the state of Fig. 4 to the state of Fig. 5), the resilience to return to the former basic form can be generated.

### Housing 40

Referring to Figs. 2 and 3 again, housing 40 includes a first housing 20 and a second housing 30, separated from each other with bending portion B of cuff band 10 as the boundary therebetween. First housing 20 and second housing 30 are arranged at one end and the other end, respectively, on the outer circumferential surface of cuff band 10, A main unit 23 of a manometer, i.e. the blood pressure measurement apparatus, is stored in first housing 20 with a display 21 formed of liquid crystal or the like and an operation unit 22 provided at the surface thereof Main components such as a control unit, an air pump, and the like are stored inside. A battery 31 qualified as a power supply is stored in second housing 30. A battery electrode 32 to be connected to battery 31 is disposed at a predetermined position in second housing 30. Electrode 32 is electrically connected to manometer main unit 23 in first housing 20 through a cable 33. The air system connection member such as a tube from the air pump in first housing 20 to air bag 11 is stored in cuff band 10.

At the site corresponding to bending portion B of cuff band 10 located between first housing 20 and second housing 30, a pivot 50 to connect first and second housings 20 and 30 in a rotatable manner is provided. At this pivot 50 is arranged a coil spring 60 to urge first and second housings 20 and 30 outwards when first and second housings 20 and 30 are rotated inwardly about pivot 50. Alternatively, pivot 50 may have coil spring 60 arranged to urge first and second housings 20 and 30 inwards when first and second housings 20 and 30 are rotated outwardly about pivot 50.

For example, in the case where the basic form corresponds to the state where inwardly folding force acts by the resilience of curled elastic member 12 per se (the form of Fig. 4), the arrangement of coil spring 60 aimed to assist that action allows the fit of cuff 10 on the wrist (biological subject) to be more firm at the time of attachment. Therefore, the squeezing performance on the subject at the time of attachment of cuff 10 can be improved. In contrast, in the case where coil spring 20 acts to broaden cuff band 10, the curled elastic member can be broadened more easily at the time of attachment to the subject since the cuff, when sphygmomanometer 100A is taken out from a case 500 that will be described afterwards, returns to the broadened state from the folded state.

### Function and Advantage

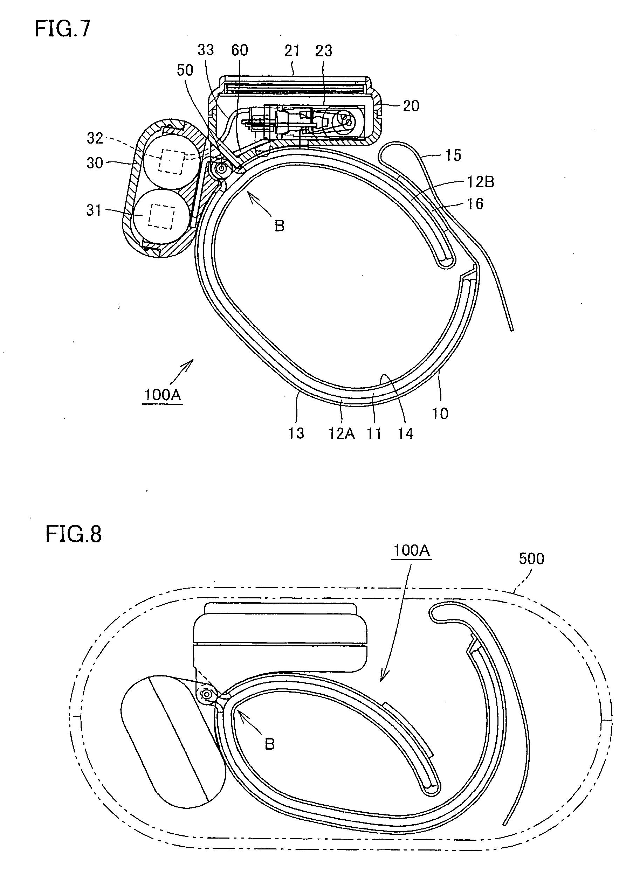

In accordance with sphygmomanometer 100A of the structure set forth above, cuff band 10 that takes a folded state as the basic form, as shown in Figs. 2 and 3, has first housing 20 and second housing 30 arranged in a separated manner with bending portion B marking the division. Therefore, cuff band 10 is bent significantly about bending portion B. As a result, the storage capability can be improved, as compared to the state of attachment shown in Figs. 6 and 7. Since the storage capability can be improved, case 500 in which sphygmomanometer 100 is stored can be made smaller, as shown in Fig. 8. Thus, portability is also improved.

The provision of regions 24 and 25 for abutment, each qualified as a stopper region, at an area of first housing 20 in the proximity of bending portion B and at an area of second housing 30 in the proximity of bending portion B allows cuffband 40 to broaden not more than a predetermined level when cuff band 40 is to be unfolded in a broadening manner (the state of Figs. 6 and 7). Since cuffband 10 can be bent about a bending point B, the storage capability can be improved even in the case where cuff band 10 incorporating a relatively rigid curled elastic member 12 is employed to facilitate a firm fit of cuff band 10 on the wrist at the time of attachment. Since it is not necessary to reduce the size of cuff band 10 or use a pliable cuff band, the squeezing performance and attachability of cuff band 10 will not be degraded.

By virtue of a structure in which manometer main unit 23 is stored in first housing 20 and battery 31 is stored in second housing 30, first housing 20 can be made thin, independent of the size of battery 31, while the size (thickness) of second housing 30 depends on the size of battery 31. Since the design of first housing 20 is not restricted by the overall height of battery 31, the size of first housing 20 and the width of cuffband 10 can be reduced. The thinness of first housing 20 is not restricted by the space for arranging battery 31. Therefore, the area of display 21 can be increased to improve visual confirmation. Even if the main unit is rendered thin, the area of operation unit 22 can be increased without being limited by the space for arranging a battery. Therefore, the operability can be improved.

### Second Embodiment

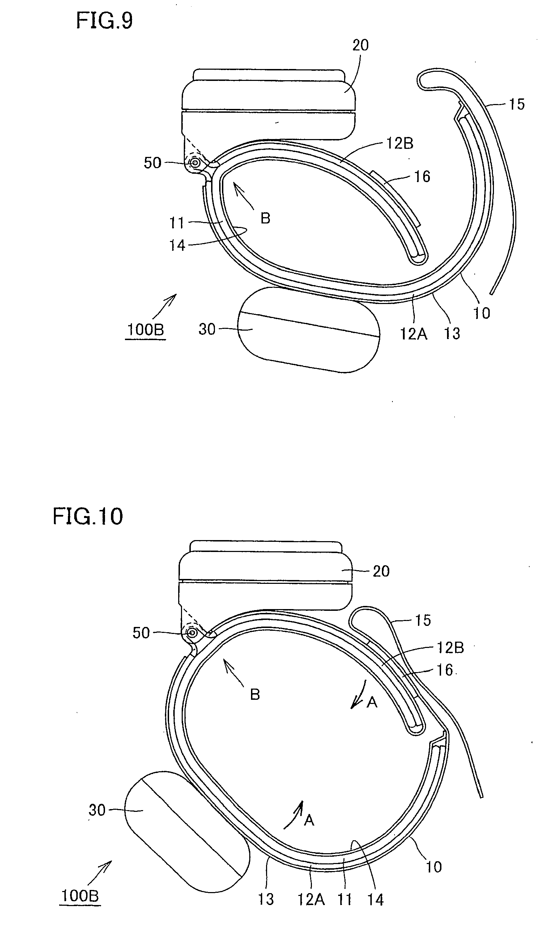

A structure of a sphygmomanometer 100B according to a second embodiment of the present invention will be described hereinafter with reference to Figs. 9 and 10. Likewise the first embodiment, the same or corresponding elements have the same reference characters allotted, and description thereof will not be repeated. Only the characteristic section will be described.

In contrast to sphygmomanometer 100A of the first embodiment having first and second housings 20 and 30 coupled in a rotatable manner about pivot 50 in housing 40, sphygmomanometer 100B of the second embodiment has second housing 30 completely separated from first housing 20. Since the connection member of the electric system such as cable 33 is stored inside cuff band 10 and electrically connected to manometer main unit 23, illustration thereof is not provided. Sphygmomanometer 100B of the second embodiment can provide advantageous effects similar to those of sphygmomanometer 100A of the first embodiment. Although pivot 50 is provided at a position corresponding to bending portion B of first housing 20, pivot 50 can be dispensed with. First housing 20 can be affixed in an independent manner on the top surface of cuff band 10, likewise second housing 30.

Although the folded state shown in Fig. 9 is taken as the basic form, a structure can be employed in which the state of being attached to a subject, as shown in Fig. 10, is taken as the basic form, corresponding to a folded state against the resilience of curled elastic member 12.

### Third Embodiment

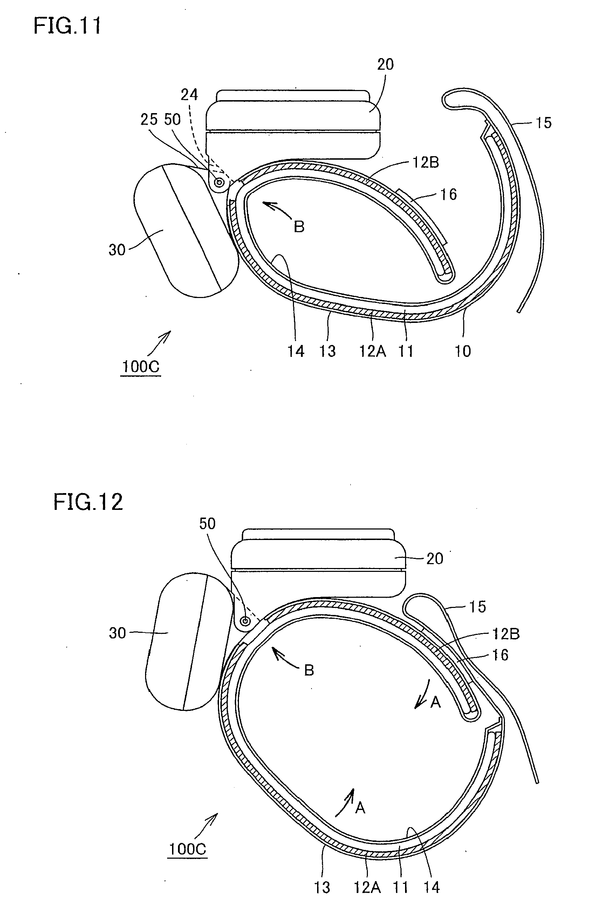

A structure of a sphygmomanometer 100C according to a third embodiment of the present invention will be described hereinafter with reference to Figs. 11 and 12. Likewise the first and second embodiments, the same or corresponding elements have the same reference characters allotted, and description thereof will not be repeated. Only the characteristic section will be described.

In contrast to sphygmomanometer 100A of the first embodiment having hinge 12C provided at curled elastic member 12 of cuff band 10, sphygmomanometer 100C of the third embodiment is absent of hinge 12C. Curled elastic member 12 of the third embodiment has first curvature portion 12A and second curvature portion 12B completely separated. Further, pivot 50 for first and second housings 20 and 30 is arranged on the upper side of the region where first curvature portion 12A and second curvature portion 12B are divided. Bending portion B is located at this region. As shown in Fig. 3, pivot 50 has coil spring 60 attached. Sphygmomanometer 100C of the third embodiment can provide advantageous effects similar to those of sphygmomanometer 100A of the first embodiment.

Although the folded state shown in Fig. 11 is taken as the basic form, a structure can be employed in which the state of being attached to a subject, as shown in Fig. 12, is taken as the basic form, corresponding to a folded state against the resilience of coil spring 60.

The above embodiments were described in which battery 31 is arranged such that the longitudinal direction thereof is orthogonal to the extending direction (wrapping direction) of cuff band 10. Battery 31 may be arranged such that the longitudinal direction thereof is identical to the extending direction of cuff band 10. Further, a structure with the function of charging the battery can be provided for battery 31 and second housing 30.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A sphygmomanometer comprising a housing (40) in which an apparatus for blood pressure measurement is stored, and a cuffband (10) supporting said housing (40), and to be wrapped around a subject, wherein
said housing (40) comprises a first housing (20) and a second housing (30),
said cuff band (10) comprises a bending portion (B) that bends,
said bending portion (B) marking a division between said first housing (20) provided at one end and said second housing (30) provided at the other end on an outer circumferential surface of said cuff band (10).

2. The sphygmomanometer according to claim 1, wherein at least one of said cuff band (10) and said housing (40) includes a resilient member to generate inwardly force when said cuff band (10) is broadened outwards about said bending portion from a folded state.

3. The sphygmomanometer according to claim 2, wherein said resilient member is a curled elastic member (12) having resilience to maintain substantially an arc shape.

4. The sphygmomanometer according to claim 3, wherein said curled elastic member (12) includes a hinge (12C) where said bending portion (B) is located.

5. The sphygmomanometer according to claim 2, wherein said resilient member is a coil spring.

6. The sphygmomanometer according to claim 1, wherein at least one of said cuff band (10) and said housing (40) includes a resilient member to generate outwardly force when said cuff band (10) is bent inwards about said bending portion from a broadened state.

7. The sphygmomanometer according to claim 6, wherein said resilient member is a curled elastic member (12) having resilience to maintain substantially an arc shape.

8. The sphygmomanometer according to claim 7, wherein said curled elastic member (12) includes a hinge (12C) where said bending portion (B) is located.

9. The sphygmomanometer according to claim 6, wherein said resilient member is a coil spring.

10. The sphygmomanometer according to claim 1, wherein said cuff band (10) comprises a curled elastic member (12A, 12B) having substantially an arc shape and separated in two, said two separated curled elastic members (12A, 12B) being arranged with said bending portion (B) therebetween, said bending portion (B) formed of a portion of said cuff band (10).

11. The sphygmomanometer according to claim 1, wherein a region for abutment is provided at an area of said first housing (20) proximate to said bending portion B and at an area of said second housing (30) proximate to said bending portion B to prevent said cuff band (40) from being broadened outwardly exceeding a predetermined level when said cuff band (40) is broadened outwardly.

12. The sphygmomanometer according to claim 1, wherein at least one of said first housing (20) and said second housing (30) is provided in a detachable manner with respect to said cuff band (20).

13. The sphygmomanometer according to claim 1, wherein
a main unit of a blood pressure measurement apparatus is stored in said first housing (20), and
a battery is stored in said second housing (30).
